(19) **Europäisches Patentamt European Patent Office Office européen des brevets**

(11) **EP 4 049 992 B1**

(12) **EUROPEAN PATENT SPECIFICATION**

(45) Date of publication and mention
of the grant of the patent:
**09.04.2025 Bulletin 2025/15**

(21) Application number: **21158864.5**

(22) Date of filing: **24.02.2021**

(51) International Patent Classification (IPC):
***C07C 51/363*** *(2006.01)* ***C07C 53/16*** *(2006.01)*

(52) Cooperative Patent Classification (CPC):
(C-Sets available)
**C07C 51/363** (Cont.)

(54) **PROCESS FOR PRODUCING MONOCHLOROACETIC ACID FROM GLYCOLIC ACID**

VERFAHREN ZUR HERSTELLUNG VON MONOCHLORESSIGSÄURE AUS GLYKOLSÄURE

PROCÉDÉ DE PRODUCTION D'ACIDE MONOCHLOROACÉTIQUE À PARTIR D'ACIDE GLYCOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.08.2022 Bulletin 2022/35**

(73) Proprietor: **Nouryon Chemicals BV
3824 BM Arnhem (NL)**

(72) Inventors:
• **AHR, Mathieu Pierre
3824 BM Arnhem (NL)**
• **VENEMAN, Rens
3824 BM Arnhem (NL)**
• **VAN LARE, Cornelis Elizabeth Johannus
3824 BM Arnhem (NL)**

• **DIRIX, Carolina Anna Maria Christina
3824 BM Arnhem (NL)**
• **RENKEMA, Eilertdina Henderika
3824 BM Arnhem (NL)**
• **PATIL, Bhaskar Sudhakar
3824 BM Arnhem (NL)**
• **TEN KATE, Antoon Jacob Berend
3824 BM Arnhem (NL)**

(74) Representative: **LKGlobal UK Ltd.
Cambridge House
Henry Street
Bath BA1 1BT (GB)**

(56) References cited:
**FR-A1- 2 575 155 JP-A- S5 344 520**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 049 992 B1

(52) Cooperative Patent Classification (CPC): (Cont.)

C-Sets
**C07C 51/363, C07C 53/16**

**Description**

The present invention is directed to a process for producing monochloroacetic acid from glycolic acid.

Background

Monochloroacetic acid (also known as chloroethanoic acid) has been produced from acetic acid and chlorine on a commercial scale for several decades. In this process, chlorine is reacted with acetic acid at elevated temperatures, via a radical reaction, to form monochloroacetic acid (MCA) and hydrogen chloride. It is well-known that this radical reaction leads to some over-chlorination, resulting in the formation of dichloroacetic acid, and to a lesser extent, depending on the conditions, trichloroacetic acid, as by-products. In many applications, compounds such as dichloroacetic acid and trichloroacetic acid are undesired, for instance, due to their regulatory status. Accordingly, to reduce the amount of dichloroacetic acid and trichloroacetic acid in the monochloroacetic acid product, dichloroacetic acid/trichloroacetic acid-containing monochloroacetic acid product mixtures are often subjected to a purification process, such as a metal-catalyzed hydrodechlorination. However, hydrodechlorination reactions require specific specialized equipment, are never fully selective and complete, and some residual by-products can be found in the final product, albeit at low levels. At the same time, additional hydrogen chloride is released by the hydrodechlorination reaction, which needs to be separated, treated and handled. As the hydrogen chloride market varies, finding an outlet for this additional hydrogen chloride can be problematic.

Accordingly, there is a need for a process for preparing a monochloroacetic acid product that contains no dichloroacetic acid or trichloroacetic acid as by-products. There is also a need for a process that uses hydrogen chloride as a reactant and chlorine source.

To this end, several publications have been issued concerning the production of monochloroacetic acid by reacting glycolic acid with hydrogen chloride.

US 4,221,921 A, for example, describes the production of monochloroacetic acid from glycolic acid by feeding glycolic acid and aqueous hydrogen chloride to a reaction zone and contacting the glycolic acid with the aqueous hydrogen chloride in the presence of hydrogen iodide catalyst at a temperature between 100 °C and 250 °C. However, the presence of the hydrogen iodide catalyst in the system will result in the formation of undesired iodo derivatives.

In a similar approach, FR 2 575 155 A1 discloses reacting glycolic acid with aqueous or anhydrous hydrogen chloride in the presence of one or more Lewis acid catalysts, other than a hydrogen halide. Again however, the presence of a Lewis acid, such as sulfuric acid, zinc chloride or even trifluoroacetic acid, is expected to complicate the downside processing of the reaction product mixture.

In a process without a catalyst, JP53044520 A discloses reacting glycolic acid, diglycolic acid, or glycolide with aqueous hydrogen chloride under batch conditions. While Example 1 reports a glycolic acid conversion rate of 82.3 mole% with 100% selectivity for monochloroacetic acid after 20 hours of stirring, upon reproducing this example, a maximum conversion of 65 mole% with a 91% selectivity for monochloroacetic acid has been found (see Comparative Example 1 below). Thus, there is a need for a process with improved conversion and/or selectivity.

It has now been found that by dosing hydrogen chloride to the glycolic acid reaction mixture in a specific manner, and at a specific temperature and pressure, one or more of the issues described above is resolved. In addition, the process has the capability of consuming hydrogen chloride and advantageously produces a monochloroacetic acid product that is inherently free of dichloroacetic acid and trichloroacetic acid.

Summary of the Disclosure

In a first aspect, the present disclosure is directed to a process for producing monochloroacetic acid from glycolic acid, the process comprising:

in an absorption step, contacting a liquid medium comprising glycolic acid and water with a hydrogen chloride-containing gas stream, resulting in the formation of a liquid medium into which hydrogen chloride has been absorbed, and
in a reaction step, reacting the glycolic acid with the absorbed hydrogen chloride at a temperature of from about 120 °C to about 180 °C and at an absolute pressure of from about 0.5 MPa to about 2 MPa, thereby providing a liquid medium comprising monochloroacetic acid,
wherein the hydrogen chloride-containing gas stream is provided during at least part of the absorption step and at least part of the reaction step in a continuous manner or intermittently in at least 2 portions.

In a second aspect, the present disclosure is directed to a process for preparing di- and trichloroacetic acid-free monochloroacetic acid, the process comprising:

in an absorption step, contacting a liquid medium comprising glycolic acid and water with a hydrogen chloride-containing gas stream, resulting in the formation of a liquid medium into which hydrogen chloride has been absorbed, in a reaction step, reacting the glycolic acid with the absorbed hydrogen chloride at a temperature of from about 120 °C to about 180 °C and at an absolute pressure of from about 0.5 MPa to about 2 MPa, thereby providing a liquid medium comprising monochloroacetic acid,

in separation step, separating the monochloroacetic acid from the liquid medium, thereby providing a first stream comprising hydrogen chloride, a second stream comprising the monochloroacetic acid and a third stream comprising glycolic acid and/or glycolic acid precursors, and

optionally, in a recycle step, recycling at least part of the first stream and/or at least part of the third stream to the absorption and/or reaction step,

wherein the hydrogen chloride-containing gas stream is provided during at least part of the absorption step and at least part of the reaction step in a continuous manner or intermittently in at least 2 portions.

Figures

Figure 1 shows the results of the conversion tests of Example 1 and Example 2, comparing batch and fed-batch conditions.

Figure 2 shows the results of the conversion tests of Examples 2-5, comparing fed-batch experiments performed at a pressure of 1.3 MPa and a temperature of 140, 150, 160 or 170 °C.

Figure 3 shows the results of the conversion tests of Examples 2, 6 and 7, comparing fed-batch experiments performed at a temperature of 150 °C and a pressure of 0.9, 1.1 or 1.3 MPa.

Detailed description

The various aspects of the present invention will be elucidated further below.

As indicated above, in a first aspect, the present invention provides a process for producing monochloroacetic acid from glycolic acid, the process comprising:

in an absorption step, contacting a liquid medium comprising glycolic acid and water with a hydrogen chloride-containing gas stream, resulting in the formation of a liquid medium into which hydrogen chloride has been absorbed, and

in a reaction step, reacting the glycolic acid with the absorbed hydrogen chloride at a temperature of from about 120 °C to about 180 °C and at an absolute pressure of from about 0.5 MPa to about 2 MPa, thereby providing a liquid medium comprising monochloroacetic acid,

wherein the hydrogen chloride-containing gas stream is provided during at least part of the absorption step and at least part of the reaction step in a continuous manner or intermittently in at least 2 portions.

Advantageously, by continuously or intermittently dosing gaseous hydrogen chloride to the hydrochlorination reaction, it has been found that the process of the first aspect of the invention can begin with a low initial water concentration. Without being bound by theory, the low initial water concentration is thought to allow, in turn, for a lower final water content in the reaction mixture, thereby favouring the forward reaction of the system (i.e., the production of monochloroacetic acid and water from the glycolic acid reacting with the absorbed hydrogen chloride) and resulting in increased yields. In addition, the low initial water content has been found to allow for a more concentrated system, and hence, a faster rate of reaction.

Furthermore, by continuously or intermittently dosing gaseous hydrogen chloride to the hydrochlorination reaction, the process of the first aspect of the invention is provided with all necessary chloride ions, such that an additional chloride source, e.g., zinc chloride or calcium chloride, is not required.

In the absorption step, a liquid medium comprising glycolic acid and water is contacted with a hydrogen chloride-containing gas stream.

The hydrogen chloride-containing gas stream is provided in a continuous manner or intermittently in at least 2, preferably at least 4, more preferably at least 6, and most preferably at least 10 portions. The duration of each portion and the time intervals between the portions may be the same or different. If many portions are added at short intervals, or if portions are provided for long durations, continuous dosing is approached.

Continuous dosing, which is the preferred manner of dosing, may be performed at constant or variable rate. The rate at which the hydrogen chloride-containing gas stream is continuously dosed is preferably controlled by an automated pressure-controlled dosing system.

Preferably, the hydrogen chloride-containing gas stream is provided to the process to maintain the reaction step at an absolute pressure of from about 0.6 MPa to about 1.7 MPa, preferably from about 0.7 MPa to about 1.5 MPa, more preferably from about 0.8 MPa to about 1.5 MPa, and even more preferably from about 0.9 MPa to about 1.3 MPa. With intermittent dosing, this may be achieved by adding a portion of the hydrogen chloride-containing gas stream when the reactor pressure is detected to fall below a predetermined threshold within the range, while with continuous dosing, the rate of addition of the hydrogen chloride-containing gas can be varied to maintain a relatively constant reactor pressure.

The hydrogen chloride-containing gas stream provided to the process may be a purified hydrogen chloride-containing gas stream, containing, for example, at least 95 vol%, preferably at least 99 vol%, of hydrogen chloride.

The contacting of the liquid medium with the hydrogen chloride-containing gas stream may be carried out in manners known in the art. In general, the mixing conditions should be sufficient to ensure intimate contact and proper mass-transfer between the hydrogen chloride-containing gas stream and the liquid medium.

As mentioned above, it is advantageous for the liquid medium to have a low initial water concentration. It has been found however, that a minimum concentration of water is needed to initiate the reaction step, for example, to dissolve the gaseous hydrogen chloride and solubilize the glycolic acid in the liquid medium. Accordingly, before contact with the hydrogen chloride-containing gas stream, water is preferably present in the liquid medium in an amount of from about 10 wt% to about 30 wt%, preferably from about 15 wt% to about 30 wt%, and more preferably from about 15 wt% to about 25 wt%, based on the total weight of the liquid medium.

In addition to water and glycolic acid, before contact with the hydrogen chloride-containing gas stream, the liquid medium may contain hydrogen chloride, preferably in an amount of from about 5 wt% to about 15 wt% and more preferably from about 10 wt% to about 15 wt%, based on the total weight of the liquid medium. In particular, the liquid medium may be a mixture of glycolic acid and aqueous hydrogen chloride. When aqueous hydrogen chloride is used in the liquid medium, it is preferably added as a solution of from 30 to 38% HCl.

Before contact with the hydrogen chloride-containing gas stream, the liquid medium may also contain glycolic acid precursors.

In the reaction step, the glycolic acid reacts with the absorbed hydrogen chloride to form monochloroacetic acid.

Preferably, the temperature of the reaction step is from about 130 °C to about 170 °C, more preferably from about 135 °C to about 160 °C, and even more preferably from about 140 °C to 155 °C. If the temperature is too high, degradation of the products and/or reactants occurs, while if the temperature is too low, the reaction is slow to proceed. For an economical process, the reaction step may, for example, be operated at about 150 °C.

As mentioned above, the absolute pressure of the reaction step is preferably from about 0.6 MPa to about 1.7 MPa, preferably from about 0.7 MPa to about 1.5 MPa, more preferably from about 0.8 MPa to about 1.5 MPa, and even more preferably from about 0.9 MPa to about 1.3 MPa. Running the reaction step under high pressure creates a driving force for the forward reaction (i.e., the production of monochloroacetic acid and water from the glycolic acid reacting with the absorbed hydrogen chloride). However, above an absolute pressure of about 2 MPa, operational constraints become limiting. For an economical process, the reaction step may, for example, be operated at an absolute pressure of about 0.9 MPa.

In contrast to US 4,221,921 A and FR 2 575 155 A1 (discussed above), the process of the present invention does not require the use of a catalyst. Accordingly, preferably, no catalyst is provided to the liquid medium before, during or after the absorption step and/or the reaction step of the present process. Furthermore, the addition of other chloride sources, such as salt chloride, e.g., $CaCl_2$, $ZnCl_2$ and LiCl, to the reaction mixture does not result in a significantly increased reaction rate. Accordingly, preferably, no other chloride source is provided to the liquid medium before, during or after the absorption step and/or the reaction step of the present process.

The reaction step is preferably carried out for a maximum time of 24 hours, preferably for less than 10 hours, and more preferably for less than 6 hours.

The process of the first aspect may further comprise:

separating the monochloroacetic acid from the liquid medium, thereby providing a first stream comprising hydrogen chloride, a second stream comprising the monochloroacetic acid and a third stream comprising glycolic acid and/or glycolic acid precursors, and

optionally, in a recycle step, recycling at least part of the first stream and/or at least part of the third stream to the absorption and/or reaction step.

In the separation step, the monochloroacetic acid may be separated from the liquid medium by thermal separation, crystallization or by extraction. Preferably, the monochloroacetic acid is separated from the liquid medium by thermal separation.

As is known in the art, in thermal separation, two or more components are separated based on their difference in relative volatility. The component with a higher volatility is selectively vaporized from a liquid mixture and subsequently condensed. Thermal separation can result in complete separation of the components present in the starting liquid mixture or partial separation resulting in concentration of selected components in the liquid mixture. Thermal separation can be done using reduced pressure or at elevated pressure. Separation performance of a thermal separation step can be enhanced by providing several theoretical equilibrium stages either by using trays, structured packing or random packing. A reflux can be used wherein part of the condensed vapors are returned in the thermal separation unit to enhance the separation performance. Examples of thermal separation processes include flash distillation, vacuum distillation, fractional distillation, short path distillation, azeotropic distillation, extractive distillation, reactive distillation, stripping and rectification.

Preferably, thermal separation is performed by distillation. Distillation can be performed in a distillation or a stripping column, or a flash distillation may be used.

The term "distillation" in this specification includes any form of distillation, including flash distillation, vacuum distillation, fractional distillation, short path distillation, azeotropic distillation, extractive distillation and reactive distillation.

The first stream of the distillation process may be an aqueous mixture comprising hydrogen chloride and water.

The second stream of the distillation process may be an aqueous mixture comprising water and the monochloroacetic acid.

The third stream of the distillation process comprises glycolic acid and/or glycolic acid precursors. The term "glycolic acid precursors" is used herein to mean any substance that reacts or hydrolyses in water to provide glycolic acid. Suitable glycolic acid precursors are typically esters and ethers of glycolic acid and/or monochloroacetic acid and include, for example, glycoloyloxy acetic acid, chloroacetoxy acetic acid and diglycolic acid.

In the recycle step, at least part of the third stream may be recycled to the absorption and/or reaction step. In addition, or alternatively, at least a part of the first stream may be recycled to the absorption and/or reaction step.

The present disclosure is further directed to a process for preparing di- and trichloroacetic acid-free monochloroacetic acid, the process comprising:

in an absorption step, contacting a liquid medium comprising glycolic acid and water with a hydrogen chloride-containing gas stream, resulting in the formation of a liquid medium into which hydrogen chloride has been absorbed, in a reaction step, reacting the glycolic acid with the absorbed hydrogen chloride at a temperature of from about 120 °C to about 180 °C and at an absolute pressure of from about 0.5 MPa to about 2 MPa, thereby providing a liquid medium comprising monochloroacetic acid, in a separation step, separating the monochloroacetic acid from the liquid medium, thereby providing a first stream comprising hydrogen chloride, a second stream comprising the monochloroacetic acid and a third stream comprising glycolic acid and/or glycolic acid precursors, and optionally, in a recycle step, recycling at least part of the first stream and/or at least part of the third stream to the absorption and/or reaction step, wherein the hydrogen chloride-containing gas stream is provided during at least part of the absorption step and at least part of the reaction step in a continuous manner or intermittently in at least 2 portions.

In the process for preparing di- and trichloroacetic acid-free monochloroacetic acid, the absorption step, reaction step, separation step and recycle step may be performed as described above in relation to the process of the first aspect.

As set out above, the herein disclosed processes comprise an absorption step and a reaction step. In the absorption step, hydrogen chloride is absorbed in the liquid reaction medium. In the reaction step, the absorbed hydrogen chloride is reacted with glycolic acid to form monochloroacetic acid and water. As mentioned above, the hydrogen chloride-containing gas stream is provided during at least part of the absorption step (for absorption) and during at least part of the reaction step (to maintain the pressure of the reaction step).

Preferably the absorption and reaction steps are carried out simultaneously. That is, it is preferred that the reaction step starts as soon as hydrogen chloride is absorbed into the liquid reaction medium in the absorption step, and that both the absorption and reaction steps continue, simultaneously, until the hydrochlorination process is terminated. If the liquid medium contains hydrogen chloride before contact with the hydrogen chloride-containing gas stream (as described above), the reaction step may start before or at the same time as the absorption step.

Accordingly, the absorption step and the reaction step of the herein disclosed processes may be performed in one batch reactor, with the liquid medium of the batch reactor varying in content of, *inter alia,* glycolic acid, water, hydrogen chloride and monochloroacetic acid as the hydrochlorination process proceeds, and the hydrogen chloride-containing gas stream maintaining the pressure requirement of the reaction step. Alternatively, the absorption and reaction steps of the herein disclosed processes may be carried out in multiple batch reactors. With either set-up, the processes may include periodic removal of the liquid medium from the batch reactor(s) for separation.

Alternatively, the absorption step and the reaction step of the herein disclosed processes may be performed in a continuously operating system in one reactor or in a cascade of continuous flow reactors, optionally with multiple feeding points. In the continuously operating system the liquid medium is continuously fed and removed from the reactor(s).

It is noted that various elements of the present invention, including but not limited to preferred ranges for the various parameters, can be combined unless they are mutually exclusive.

The invention will be elucidated by the following examples without being limited thereto or thereby.

Examples

In the following examples, the composition of the reaction mixture has been determined by liquid chromatography analysis. All values in the tables are expressed in weight % compared to the total mass of the liquid phase. The time has been chosen with t = 0 being the time at which the reaction mixture reached the desired temperature.

The following abbreviations are used:

GA = Glycolic acid;
MCA = Monochloroacetic acid;
GAA = (Glycoloyloxy)acetic acid;
CAAA = Chloroacetoxyacetic acid;
Trimer-OH = 2-(2'-hydroxyacetoxy)acetoxyacetic acid.

Dichloroacetic acid and diglycolic acid were not detected in any of the experiments.

The conversion (of GA to MCA), as expressed in the tables, has been calculated using the formulas:

$$Conv.\,(\%) = 100 \times \frac{MCA\ units}{(MCA\ units + GA\ units)}$$

$$MCA\ units = [MCA]/MW_{MCA} + 2 \times [CAAA]/MW_{CAAA}$$

$$GA\ units = [GA]/MW_{GA} + 2 \times [GAA]/MW_{GAA} + 3 \times [Trimer-OH]/MW_{Trimer-OH}$$

where $[X]$ = weight % of the compound X compared to the total mass of the liquid phase determined by liquid chromatography analysis and $MW_x$ = molar mass of the compound X.

**Comparative Example 1: Reaction of GA with HCl at 150 °C under batch conditions (based on JP53044520 A, Example 1)**

A double wall corrosion-resistant reactor equipped with a mechanical stirrer, a gas dosing inlet, a sample outlet (in the liquid mixture in the reactor) and a bottom valve was loaded with GA (145.7 g; MW 76.05 g.mol$^{-1}$; 1.9 mol; 1.0 equiv.) and HCl-36% (500.0 g; MW 36.46 g.mol$^{-1}$; 4.9 mol; 2.6 equiv.). The reactor was then closed, and the mixture was heated while stirring (500 rpm) to the desired temperature (150 °C) using a circulating oil bath; the pressure increased to a maximal level of 1.6 MPa. The mixture was maintained at the desired temperature for a predetermined time while samples were taken for future analyses. At the end of the test, the reaction mixture was cooled to RT, the overpressure was released, and the content of the reactor was transferred to a glass bottle, weighed and analyzed. The results are shown in Table 1.

*Table* 1: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| 15 | 8.2 | 19.6 | 2.3 | 3.3 | 0.3 | 0.0 |
| 75 | 34.4 | 14.2 | 9.6 | 1.4 | 0.7 | 0.2 |
| 155 | 49.4 | 10.8 | 13.7 | 0.6 | 0.6 | 0.4 |
| 240 | 56.8 | 9.2 | 15.8 | 0.5 | 0.6 | 0.2 |
| 570 | 61.5 | 7.5 | 18.0 | 0.2 | 0.5 | 0.8 |
| 755 | 63.2 | 7.3 | 18.2 | 0.3 | 0.5 | 0.6 |
| 1225 | 64.5 | 7.2 | 18.1 | 0.2 | 0.5 | 0.6 |

As can be seen from Table 1, the selectivity of this reaction for MCA (calculated using the formula ([$MCA$]/$MW_{M-}$

$_{CA}$)/ ([MCA]/$MW_{MCA}$ + 2 × [GAA]/$MW_{GAA}$ + 2 × [CAAA]/$MW_{CAAA}$ + 3 × [Trimer - OHJ/$MW_{Trimer-OH}$)) is well below the 100% reported in JP 53044520 A. Moreover, the large amount of water introduced in the system (well above 35 % of the initial reaction mixture) results in a maximal conversion level of ca. 65%, which is significantly lower than the values reported in the Japanese document.

Without being bound by theory, this discrepancy in results is thought to be due to the distillation step used in the Examples of JP53044520 A, which could have led to further reaction and overestimated conversion levels. It may also have led the inventors to conclude that the reaction was fully selective to MCA. However, in-depth analysis of the reproduced product mixture shows that multiple products are formed, such as GA and MCA-based esters.

## Example 2: Reaction of GA with HCl under fed-batch conditions at 150 °C and 1.3 MPa

A double wall corrosion-resistant reactor equipped with a mechanical stirrer, a gas dosing inlet, a sample outlet (in the liquid mixture in the reactor) and a bottom valve was loaded with GA (357.3 g; MW 76.05 g.mol$^{-1}$; 4.7 mol; 1.0 equiv.) and a predetermined amount of HCl-29.5% (150.0 g; MW 36.46 g.mol$^{-1}$; 1.2 mol; 0.26 equiv.). The reactor was then closed and the mixture was heated while stirring (500 rpm) to the desired temperature using a circulating oil bath. When the mixture had reached the desired temperature (150 °C) HCl-gas was added via a flowmeter, with a maximum rate of 4.0 g HCl / min until the predetermined target pressure (here 1.3 MPa) was reached. The pressure was then maintained at the target value (here about 1.3 MPa) by dosing HCl-gas as required over the course of the reaction while keeping the temperature constant. Samples were taken over time for future analysis.

At the end of the test, the reaction mixture was cooled to RT, the overpressure was released, and the content of the reactor was transferred to a glass bottle, weighed and analyzed. The results are shown in Table 2.

*Table* 2: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| **15** | 9.7 | 36.5 | 5.2 | 13.6 | 1.7 | 2.0 |
| **30** | 15.4 | 34.3 | 8.2 | 11.7 | 2.4 | 1.6 |
| **60** | 25.5 | 31.1 | 13.8 | 9.1 | 3.5 | 1.1 |
| **121** | 39.6 | 25.9 | 21.7 | 6.0 | 4.4 | 0.6 |
| **240** | 55.8 | 19.3 | 31.0 | 3.2 | 4.5 | 0.2 |
| **420** | 66.3 | 14.8 | 37.0 | 2.1 | 4.3 | 0.1 |
| **670** | 75.5 | 10.7 | 42.7 | 1.3 | 3.5 | 0.1 |
| **970** | 82.8 | 7.4 | 46.4 | 0.8 | 2.8 | 0.0 |
| **1200** | 85.6 | 6.4 | 48.9 | 0.6 | 2.5 | 0.0 |
| **1465** | 87.3 | 5.6 | 49.2 | 0.4 | 2.2 | 0.0 |
| **1685** | 88.0 | 5.3 | 49.7 | 0.4 | 2.1 | 0.0 |

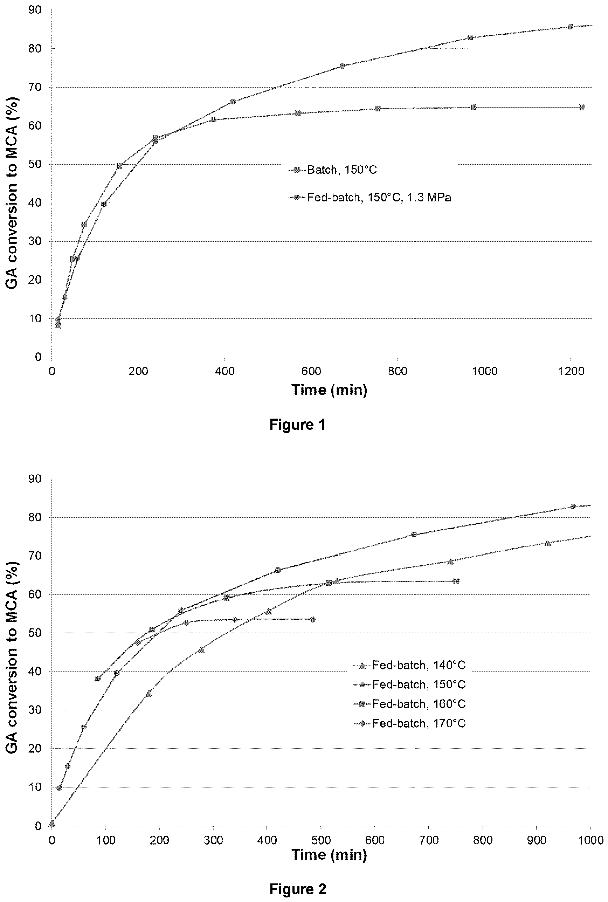

Examples 1 and 2, together with corresponding Figure 1, show that using a limited amount of water at the start of the reaction (i.e., 20 wt% in Example 2 vs 50 wt% in Example 1) and applying continuous **HCl** gas dosing has an advantageous effect on the maximal reachable conversion level, which is increased from ca. 65% to ca. 85% after 1200 min of reaction.

## Example 3: Reaction of GA with HCl under fed-batch conditions at 140 °C and 1.3 MPa

In this example, the procedure described in Example 2 was applied at 140 °C and 1.3 MPa. The results are shown in Table 3.

*Table* 3: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| **0** | 0.8 | 46.2 | 0.5 | 15.0 | 0.1 | 1.8 |
| **180** | 34.4 | 27.2 | 18.2 | 7.4 | 4.5 | 0.8 |
| **278** | 45.9 | 22.4 | 24.1 | 5.2 | 5.1 | 0.5 |

(continued)

| Time (min) | Conv. (%) | GA (%) | MCA (%) | GAA (%) | CAAA (%) | Trimer-OH (%) |
|---|---|---|---|---|---|---|
| 402 | 55.8 | 18.4 | 29.2 | 3.6 | 5.2 | 0.3 |
| 530 | 63.6 | 15.4 | 33.9 | 2.5 | 4.9 | 0.2 |
| 740 | 68.7 | 13.3 | 36.8 | 1.9 | 4.6 | 0.1 |
| 920 | 73.4 | 11.2 | 39.3 | 1.4 | 4.2 | 0.1 |
| 1260 | 80.3 | 8.5 | 43.7 | 0.8 | 3.2 | 0.0 |

**Example 4: Reaction of GA with HCl under fed-batch conditions at 160 °C and 1.3 MPa**

In this example, the procedure described in Example 2 was applied at 160 °C and 1.3 MPa. The results are shown in Table 4.

*Table* 4: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| 90 | 38.2 | 28.6 | 22.6 | 5.9 | 4.0 | 0.5 |
| 190 | 50.9 | 23.0 | 30.3 | 3.7 | 4.1 | 0.2 |
| 320 | 59.0 | 19.2 | 35.7 | 2.8 | 3.7 | 0.2 |
| 520 | 62.9 | 17.3 | 38.3 | 2.4 | 3.3 | 0.1 |
| 750 | 63.4 | 16.9 | 38.9 | 2.4 | 3.2 | 0.1 |

**Example 5: Reaction of GA with HCl under fed-batch conditions at 170 °C and 1.3 MPa**

In this example, the procedure described in Example 2 was applied at 170 °C and 1.3 MPa. The results are shown in Table 5.

*Table* 5: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| 160 | 47.5 | 26.5 | 30.7 | 4.3 | 4.0 | 0.3 |
| 250 | 52.6 | 22.5 | 32.4 | 3.5 | 3.6 | 0.2 |
| 340 | 53.5 | 22.1 | 33.3 | 3.6 | 3.5 | 0.2 |
| 490 | 53.6 | 21.5 | 33.3 | 3.9 | 3.5 | 0.2 |

The content of the reactor collected at the end of this test exhibited a much stronger discoloration (dark brown to black liquid) than all other experiments performed at lower temperatures (usually pale yellow to yellow liquid) and contained some solid black material not observed in other tests.

The content of the headspace of the reactor was analyzed at the end of the experiment by means of GC-MS and FT-IR. In addition to the expected hydrogen chloride, the presence of a significant amount of carbon monoxide, along with trace amounts of methyl chloride and carbon dioxide, was detected, indicating the degradation of some reagent and/or products.

Examples 2-5, together with corresponding Figure 2, show that the reaction temperature has an effect on the rate of the reaction, with the best conversions at 140 °C and 150 °C.

**Example 6: Reaction of GA with HCl under fed-batch conditions at 150 °C and 1.1 MPa**

In this example, the procedure described in Example 2 was applied at 150 °C and 1.1 MPa. The results are shown in Table 6.

Table 6: Results of GA conversion reaction vs time

| Time (min) | Conv. (%) | GA (%) | MCA (%) | GAA (%) | CAAA (%) | Trimer-OH (%) |
|---|---|---|---|---|---|---|
| 15 | 10.4 | 37.5 | 5.6 | 12.1 | 1.7 | 1.6 |
| 30 | 14.5 | 36.0 | 7.8 | 11.0 | 2.2 | 1.4 |
| 60 | 22.5 | 33.3 | 12.4 | 8.9 | 3.0 | 1.0 |
| 120 | 34.5 | 28.2 | 18.8 | 6.4 | 3.9 | 0.6 |
| 240 | 49.3 | 22.1 | 27.2 | 3.9 | 4.2 | 0.3 |
| 480 | 65.6 | 14.9 | 36.2 | 2.0 | 3.7 | 0.1 |
| 995 | 78.9 | 8.9 | 42.5 | 1.0 | 3.1 | 0.0 |
| 1490 | 82.0 | 7.6 | 45.1 | 0.8 | 2.4 | 0.0 |

## Example 7: Reaction of GA with HCl under fed-batch conditions at 150 °C and 0.9 MPa

In this example, the procedure described in Example 2 was applied at 150°C and 0.9 MPa. The results are shown in Table 7.

Table 7: Results of GA conversion reaction vs time

| Time (min) | Conv. (%) | GA (%) | MCA (%) | GAA (%) | CAAA (%) | Trimer-OH (%) |
|---|---|---|---|---|---|---|
| 15 | 7.6 | 40.3 | 4.3 | 12.8 | 1.2 | 1.6 |
| 30 | 10.9 | 38.4 | 6.5 | 11.6 | 1.7 | 4.4 |
| 60 | 18.4 | 36.1 | 10.5 | 9.6 | 2.5 | 1.1 |
| 120 | 28.4 | 32.3 | 16.4 | 7.2 | 3.2 | 0.7 |
| 240 | 40.9 | 27.1 | 23.7 | 4.7 | 3.7 | 0.4 |
| 490 | 55.5 | 19.8 | 31.7 | 3.0 | 3.9 | 0.2 |
| 990 | 69.6 | 13.2 | 39.1 | 1.6 | 3.2 | 0.1 |
| 1500 | 75.7 | 10.5 | 42.5 | 1.2 | 2.8 | 0.1 |

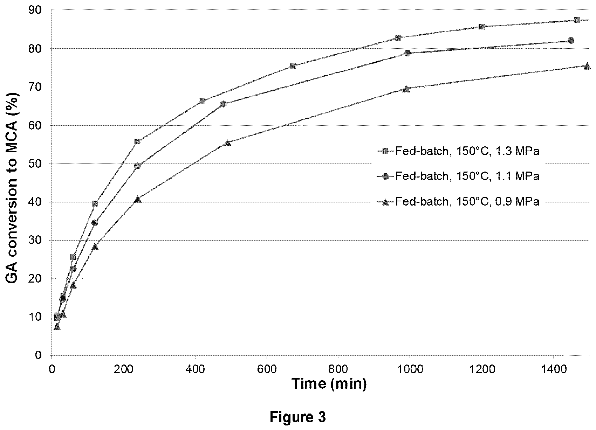

Examples 2, 6 and 7, together with corresponding Figure 3, show that the **HCl** pressure has an effect on the rate of the reaction. However, this effect is not linear and is expected to be less pronounced at high pressures (above 2.0 MPa). The additional costs and limitations of the current state of the art available equipment required to operate under these conditions are therefore expected to make this option less commercially attractive.

## Example 8: Reaction of GA in 1,4-dioxane with HCl gas at 1.3 MPa at 140 °C in the (initial) absence of water

A double wall corrosion-resistant reactor equipped with a mechanical stirrer, a gas dosing inlet, a sample outlet (in the liquid mixture in the reactor) and a bottom valve was loaded with GA (80.0 g; MW 76.05 g.mol$^{-1}$; 4.7 mol; 1.0 equiv.) and 1,4-dioxane (609.0 g; MW 88.11 g.mol$^{-1}$; 6.9 mol; 6.6 equiv.). The reactor was then closed, and the mixture was heated while stirring (500 rpm) to the desired temperature (140 °C) using a circulating oil bath.

When the mixture had reached the desired temperature (140 °C) HCl-gas was added via a flowmeter, with a maximum rate of 4.0 g **HCl** / min until the target pressure (1.3 MPa) was reached. The pressure was then maintained at about 1.3 MPa by dosing HCl-gas as required over the course of the reaction while keeping the temperature constant. Samples were taken over time for future analysis.

After 440 min reaction time at 140 °C, the reaction mixture was cooled to RT, the overpressure was released and the content of the reactor was transferred to a glass bottle, weighed and analyzed. The liquid chromatography analyses showed the absence of MCA in the reaction mixture, even in the final reaction mixture; this result was confirmed by $^{1}$H NMR analysis of the reaction mixture.

**Example 9: Reaction of GA with HCl under fed-batch conditions at 140 °C and 1.3 MPa, starting from an aqueous solution of GA**

A double wall corrosion-resistant reactor equipped with a mechanical stirrer, a gas dosing inlet, a sample outlet (in the liquid mixture in the reactor) and a bottom valve was loaded with GA (357.3 g; MW 76.05 g.mol$^{-1}$; 4.7 mol; 1.0 equiv.) and water (108.8 g; MW 18.01 g.mol$^{-1}$; 5.9 mol; 1.26 equiv.). The reactor was then closed and the mixture was heated while stirring (500 rpm) to the desired temperature using a circulating oil bath. When the mixture had reached 140 °C, HCl-gas was added via a flowmeter with a maximum rate of 4.0 g HCl / min until the pressure of 1.3 MPa was reached. The pressure was then maintained at about 1.3 MPa by dosing HCl-gas as required over the course of the reaction while keeping the temperature constant. Samples were taken over time for future analysis.

At the end of the test, the reaction mixture was cooled down to RT, the overpressure was released, and the content of the reactor was transferred to a glass bottle, weighed and analyzed. The results are shown in Table 9.

*Table* 9: *Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| 15 | 2.3 | 41.42 | 1.23 | 15.77 | 0.44 | 2.38 |
| 30 | 6.7 | 37.58 | 3.50 | 14.61 | 1.25 | 2.28 |
| 60 | 13.9 | 34.77 | 7.40 | 12.52 | 2.32 | 1.80 |
| 120 | 25.5 | 30.17 | 13.45 | 9.39 | 3.66 | 1.17 |
| 240 | 41.6 | 24.38 | 22.08 | 5.76 | 4.72 | 0.56 |
| 410 | 55.7 | 19.12 | 30.37 | 3.31 | 4.75 | 0.24 |

After a brief induction time, corresponding to the adsorption of the gaseous **HCl** in the liquid phase, the rate of reaction is the same than in Example 3, evidencing that both procedures can be equally applied.

**Example 10: Reaction of GA with HCl under fed-batch conditions at 150 °C and 1.2 MPa in the presence of zinc chloride**

A double wall corrosion-resistant reactor equipped with a mechanical stirrer, a gas dosing inlet, a sample outlet (in the liquid mixture in the reactor) and a bottom valve was loaded with GA (357.3 g; MW 76.05 g.mol$^{-1}$; 4.7 mol; 1.0 equiv.), a predetermined amount of HCl-29.5% (150.0 g; MW 36.46 g.mol$^{-1}$; 1.2 mol; 0.26 equiv.), the required amounts of water (2.9 g; MW 18.01 g.mol$^{-1}$; 0.2 mol; 0.05 equiv.) and zinc chloride (14.5 g; MW 136,315 g.mol$^{-1}$; 0.1 mol; 0.05 equiv.; 2.8 wt%). The reactor was then closed, and the mixture was heated while stirring (500 rpm) to the desired temperature (150 °C) using a circulating oil bath. When the mixture had reached the desired temperature (150 °C) HCl-gas was added via a flowmeter, with a maximum rate of 4.0 g **HCl** / min until the predetermined pressure (1.2 MPa) was reached. The pressure was then maintained at about 1.2 MPa by dosing HCl-gas as required over the course of the reaction while keeping the temperature constant. Samples were taken over time for future analysis.

At the end of the test, the reaction mixture was cooled to RT, the overpressure was released, and the content of the reactor was transferred to a glass bottle, weighed and analyzed. The results are shown in Table 10.

*Table 10: Results* of GA *conversion reaction vs time*

| Time (min) | Conv. (%) | GA (%) | MCA (%) | GAA (%) | CAAA (%) | Trimer-OH (%) |
|---|---|---|---|---|---|---|
| 15 | 6.3 | 38.3 | 3.3 | 13.5 | 1.1 | 2.0 |
| 30 | 11.8 | 35.9 | 6.1 | 11.6 | 1.9 | 1.6 |
| 60 | 20.4 | 34.0 | 11.1 | 9.0 | 2.8 | 1.1 |
| 120 | 33.8 | 27.6 | 17.8 | 6.8 | 4.1 | 0.7 |
| 240 | 51.5 | 20.5 | 27.3 | 3.6 | 4.5 | 0.3 |
| 450 | 65.1 | 15.0 | 35.3 | 2.0 | 4.1 | 0.1 |
| 670 | 74.3 | 10.8 | 39.8 | 1.3 | 3.6 | 0.1 |
| 970 | 82.2 | 7.4 | 43.9 | 0.7 | 2.7 | 0.0 |

From Table 10 it can be seen that the addition of $ZnCl_2$ to the reaction mixture does not result in an increased reaction rate under the same reaction conditions (cf. Example 2). The same result was obtained with LiCl and $CaCl_2$.

Whilst the invention has been described with reference to an exemplary embodiment, it will be appreciated that various modifications are possible within the scope of the invention.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than to mean 'consisting of'.

**Claims**

1. A process for producing monochloroacetic acid from glycolic acid, the process comprising:

   in an absorption step, contacting a liquid medium comprising glycolic acid and water with a hydrogen chloride-containing gas stream, resulting in the formation of a liquid medium into which hydrogen chloride has been absorbed, and
   in a reaction step, reacting the glycolic acid with the absorbed hydrogen chloride at a temperature of from about 120 °C to about 180 °C and at an absolute pressure of from about 0.5 **MPa** to about 2 **MPa,** thereby providing a liquid medium comprising monochloroacetic acid,
   wherein the hydrogen chloride-containing gas stream is provided during at least part of the absorption step and at least part of the reaction step in a continuous manner or intermittently in at least 2 portions.

2. A process as claimed in claim 1, wherein the temperature of the reaction step is from about 130 °C to about 170 °C, preferably from about 135 °C to about 160 °C, and more preferably from about 140 °C to 155 °C.

3. A process as claimed in claim 1 or claim 2, wherein the absolute pressure of the reaction step is from about 0.6 MPa to about 1.7 MPa, preferably from about 0.7 MPa to about 1.5 MPa, more preferably from about 0.8 MPa to about 1.5 MPa, and even more preferably from about 0.9 MPa to 1.3 MPa.

4. A process as claimed in any preceding claim, wherein, before contact with the hydrogen chloride-containing gas stream in the absorption step, water is present in the liquid medium in an amount of from about 10 wt% to about 30 wt%, preferably from about 15 wt% to about 30 wt%, and more preferably from about 15 wt% to about 25 wt%, based on the total weight of the liquid medium.

5. A process as claimed in any preceding claim, wherein the hydrogen chloride-containing gas stream is provided in a continuous manner during the process.

6. A process as claimed in any preceding claim, wherein the hydrogen chloride-containing gas stream is provided intermittently in at least 10 portions during the process.

7. A process as claimed in any preceding claim, wherein the reaction step is carried out for a maximum time of 24 hours, preferably for less than 10 hours, and more preferably for less than 6 hours.

8. A process as claimed in any preceding claim, further comprising

   in a separation step, separating the monochloroacetic acid from the liquid medium, thereby providing a first stream comprising hydrogen chloride, a second stream comprising the monochloroacetic acid and a third stream comprising glycolic acid and/or glycolic acid precursors, and
   optionally, in a recycle step, recycling at least part of the first stream and/or at least part of the third stream to the absorption and/or reaction step.

9. A process as claimed in claim 1 for preparing di- and trichloroacetic acid-free monochloroacetic acid from glycolic acid, the process comprising:

   in an absorption step, contacting a liquid medium comprising glycolic acid and water with a hydrogen chloride-containing gas stream, resulting in the formation of a liquid medium into which hydrogen chloride has been absorbed,

in a reaction step, reacting the glycolic acid with the absorbed hydrogen chloride at a temperature of from about 120 °C to about 180 °C and at an absolute pressure of from about 0.5 **MPa** to about 2 **MPa,** thereby providing a liquid medium comprising monochloroacetic acid,

in a separation step, separating the monochloroacetic acid from the liquid medium, thereby providing a first stream comprising hydrogen chloride, a second stream comprising the monochloroacetic acid and a third stream comprising glycolic acid and/or glycolic acid precursors, and

optionally, in a recycle step, recycling at least part of the first stream and/or at least part of the third stream to the absorption and/or reaction step,

wherein the hydrogen chloride-containing gas stream is provided during at least part of the absorption step and at least part of the reaction step in a continuous manner or intermittently in at least 2 portions.

10. A process as claimed in any preceding claim, wherein the absorption step and the reaction step are performed in one batch reactor, and the hydrogen chloride-containing gas stream maintains the pressure requirement of the reaction step.

**Patentansprüche**

1. Verfahren zur Herstellung von Monochloressigsäure aus Glykolsäure, wobei das Verfahren Folgendes umfasst:

in einem Absorptionsschritt, Kontaktieren eines flüssigen Mediums, das Glykolsäure und Wasser umfasst, mit einem Chlorwasserstoff enthaltenden Gasstrom, was zur Bildung eines flüssigen Mediums führt, in das Chlorwasserstoff absorbiert worden ist, und

in einem Reaktionsschritt, Reagieren der Glykolsäure mit dem absorbierten Chlorwasserstoff bei einer Temperatur von etwa 120 °C bis etwa 180 °C und bei einem absoluten Druck von etwa 0,5 MPa bis etwa 2 MPa, wodurch ein flüssiges Medium bereitgestellt wird, das Monochloressigsäure umfasst,

wobei der Chlorwasserstoff enthaltende Gasstrom während mindestens eines Teils des Absorptionsschritts und mindestens eines Teils des Reaktionsschritts auf kontinuierliche Weise oder zeitweise in mindestens 2 Portionen bereitgestellt wird.

2. Verfahren nach Anspruch 1, wobei die Temperatur des Reaktionsschritts etwa 130 °C bis etwa 170 °C, bevorzugt etwa 135 °C bis etwa 160 °C und noch bevorzugter etwa 140 °C bis 155 °C beträgt.

3. Verfahren nach Anspruch 1 oder Anspruch 2, wobei der absolute Druck des Reaktionsschritts etwa 0,6 MPa bis etwa 1,7 MPa, bevorzugt etwa 0,7 MPa bis etwa 1,5 MPa, noch bevorzugter etwa 0,8 MPa bis etwa 1,5 MPa und sogar noch bevorzugter etwa 0,9 MPa bis 1,3 MPa beträgt.

4. Verfahren nach einem vorhergehenden Anspruch, wobei vor Kontakt mit dem Chlorwasserstoff enthaltenden Gasstrom im Absorptionsschritt, Wasser in dem flüssigen Medium in einer Menge von etwa 10 Gew.-% bis etwa 30 Gew.-%, bevorzugt etwa 15 Gew.-% bis etwa 30 Gew.-% und noch bevorzugter etwa 15 Gew.-% bis etwa 25 Gew.-%, auf das Gesamtgewicht des flüssigen Mediums bezogen, vorliegt.

5. Verfahren nach einem vorhergehenden Anspruch, wobei der Chlorwasserstoff enthaltende Gasstrom während des Verfahrens auf kontinuierliche Weise bereitgestellt wird.

6. Verfahren nach einem vorhergehenden Anspruch, wobei der Chlorwasserstoff enthaltende Gasstrom während des Verfahrens zeitweilig in mindestens 10 Portionen bereitgestellt wird.

7. Verfahren nach einem vorhergehenden Anspruch, wobei der Reaktionsschritt während einer maximalen Zeitdauer von 24 Stunden, bevorzugt weniger als 10 Stunden und noch bevorzugter weniger als 6 Stunden ausgeführt wird.

8. Verfahren nach einem vorhergehenden Anspruch, ferner Folgendes umfassend

in einem Trennungsschritt, Trennen der Monochloressigsäure von dem flüssigen Medium, wodurch ein erster Strom, der Chlorwasserstoff umfasst, ein zweiter Strom, der Monochloressigsäure umfasst, und ein dritter Strom, der Glykolsäure und/oder Glykolsäurevorläufer umfasst, bereitgestellt wird, und

wahlweise, in einem Rückführschritt, Rückführen mindestens eines Teils des ersten Stroms und/oder mindestens eines Teils des dritten Stroms zum Absorptions- und/oder Reaktionsschritt.

9. Verfahren nach Anspruch 1 zur Herstellung von von Di- und Trichloressigsäure freier Monochloressigsäure aus Glykolsäure, wobei das Verfahren Folgendes umfasst:

in einem Absorptionsschritt, Kontaktieren eines flüssigen Mediums, das Glykolsäure und Wasser umfasst, mit einem Chlorwasserstoff enthaltenden Gasstrom, was zur Bildung eines flüssigen Mediums führt, in das Chlorwasserstoff absorbiert worden ist,

in einem Reaktionsschritt, Reagieren der Glykolsäure mit dem absorbierten Chlorwasserstoff bei einer Temperatur von etwa 120 °C bis etwa 180 °C und bei einem absoluten Druck von etwa 0,5 MPa bis etwa 2 MPa, wodurch ein flüssiges Medium bereitgestellt wird, das Monochloressigsäure umfasst,

in einem Trennungsschritt, Trennen der Monochloressigsäure von dem flüssigen Medium, wodurch ein erster Strom, der Chlorwasserstoff umfasst, ein zweiter Strom, der Monochloressigsäure umfasst, und ein dritter Strom, der Glykolsäure und/oder Glykolsäurevorläufer umfasst, bereitgestellt wird, und

wahlweise, in einem Rückführschritt, Rückführen mindestens eines Teils des ersten Stroms und/oder mindestens eines Teils des dritten Stroms zum Absorptions- und/oder Reaktionsschritt,

wobei der Chlorwasserstoff enthaltende Gasstrom während mindestens eines Teils des Absorptionsschritts und mindestens eines Teils des Reaktionsschritts auf kontinuierliche Weise oder zeitweilig in mindestens 2 Portionen bereitgestellt wird.

10. Verfahren nach einem vorhergehenden Anspruch, wobei der Absorptionsschritt und der Reaktionsschritt in einem Chargenreaktor ausgeführt werden und der Chlorwasserstoff enthaltende Gasstrom das Druckerfordernis des Reaktionsschritts aufrechterhält.

**Revendications**

1. Procédé de production d'acide monochloroacétique à partir d'acide glycolique, le procédé comprenant :

dans une étape d'absorption, la mise en contact d'un milieu liquide comprenant de l'acide glycolique et de l'eau avec un flux gazeux contenant du chlorure d'hydrogène, entraînant la formation d'un milieu liquide dans lequel le chlorure d'hydrogène a été absorbé, et

dans une étape de réaction, la réaction de l'acide glycolique avec le chlorure d'hydrogène absorbé à une température d'environ 120 °C à environ 180 °C et à une pression absolue d'environ 0,5 MPa à environ 2 MPa, fournissant ainsi un milieu liquide comprenant l'acide monochloroacétique,

dans lequel le flux gazeux contenant du chlorure d'hydrogène est fourni pendant au moins une partie de l'étape d'absorption et au moins une partie de l'étape de réaction d'une manière continue ou par intermittence en au moins 2 portions.

2. Procédé selon la revendication 1, dans lequel la température de l'étape de réaction est d'environ 130 °C à environ 170 °C, de préférence d'environ 135 °C à environ 160 °C, et plus préférablement d'environ 140 °C à 155 °C.

3. Procédé selon la revendication 1 ou la revendication 2, dans lequel la pression absolue de l'étape de réaction est d'environ 0,6 MPa à environ 1,7 MPa, de préférence d'environ 0,7 MPa à environ 1,5 MPa, plus préférablement d'environ 0,8 MPa à environ 1,5 MPa, et encore plus préférablement d'environ 0,9 MPa à 1,3 MPa.

4. Procédé selon une quelconque revendication précédente, dans lequel, avant la mise en contact avec le flux gazeux contenant du chlorure d'hydrogène dans l'étape d'absorption, de l'eau est présente dans le milieu liquide en une quantité d'environ 10 % en poids à environ 30 % en poids, de préférence d'environ 15 % en poids à environ 30 % en poids, et plus préférablement d'environ 15 % en poids à environ 25 % en poids, sur la base du poids total du milieu liquide.

5. Procédé selon une quelconque revendication précédente, dans lequel le flux gazeux contenant du chlorure d'hydrogène est fourni d'une manière continue pendant le procédé.

6. Procédé selon une quelconque revendication précédente, dans lequel le flux gazeux contenant du chlorure d'hydrogène est fourni par intermittence en au moins 10 portions pendant le procédé.

7. Procédé selon une quelconque revendication précédente, dans lequel l'étape de réaction est réalisée pendant une durée maximale de 24 heures, de préférence pendant moins de 10 heures, et plus préférablement pendant moins de 6 heures.

8. Procédé selon une quelconque revendication précédente, comprenant en outre

dans une étape de séparation, la séparation de l'acide monochloroacétique du milieu liquide, fournissant ainsi un premier flux comprenant le chlorure d'hydrogène, un deuxième flux comprenant l'acide monochloroacétique et un troisième flux comprenant l'acide glycolique et/ou des précurseurs d'acide glycolique, et éventuellement, dans une étape de recyclage, le recyclage d'au moins une partie du premier flux et/ou d'au moins une partie du troisième flux vers l'étape d'absorption et/ou de réaction.

9. Procédé selon la revendication 1 permettant de préparer de l'acide monochloroacétique exempt d'acide di- et trichloroacétique à partir d'acide glycolique, le procédé comprenant :

dans une étape d'absorption, la mise en contact d'un milieu liquide comprenant de l'acide glycolique et de l'eau avec un flux gazeux contenant du chlorure d'hydrogène, entraînant la formation d'un milieu liquide dans lequel le chlorure d'hydrogène a été absorbé,
dans une étape de réaction, la réaction de l'acide glycolique avec le chlorure d'hydrogène absorbé à une température d'environ 120 °C à environ 180 °C et à une pression absolue d'environ 0,5 MPa à environ 2 MPa, fournissant ainsi un milieu liquide comprenant l'acide monochloroacétique,
dans une étape de séparation, la séparation de l'acide monochloroacétique du milieu liquide, fournissant ainsi un premier flux comprenant le chlorure d'hydrogène, un deuxième flux comprenant l'acide monochloroacétique et un troisième flux comprenant l'acide glycolique et/ou des précurseurs d'acide glycolique, et éventuellement, dans une étape de recyclage, le recyclage d'au moins une partie du premier flux et/ou d'au moins une partie du troisième flux vers l'étape d'absorption et/ou de réaction,
dans lequel le flux gazeux contenant du chlorure d'hydrogène est fourni pendant au moins une partie de l'étape d'absorption et au moins une partie de l'étape de réaction d'une manière continue ou par intermittence en au moins 2 portions.

10. Procédé selon une quelconque revendication précédente, dans lequel l'étape d'absorption et l'étape de réaction sont réalisées dans un réacteur discontinu, et le flux gazeux contenant du chlorure d'hydrogène maintient les critères requis de pression de l'étape de réaction.

Figure 1

Figure 2

Figure 3

**EP 4 049 992 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4221921 A
- FR 2575155 A1
- JP 53044520 A